# EUROPÄISCHE PATENTSCHRIFT

(11) **EP 0 817 826 B1**
(45) Veröffentlichungstag und Bekanntmachung des Hinweises auf die Patenterteilung: **24.05.2000**
(21) Anmeldenummer: 96908084.5
(22) Anmeldetag: 20.03.1996
(51) Int. Cl.: C11D 1/825, C11D 1/835, B01F 17/00, A61K 7/50

(54) **NIEDRIGVISKOSE TRÜBUNGSMITTELKONZENTRATE**
LOW-VISCOSITY OPACIFYING AGENT CONCENTRATES
CONCENTRES D'AGENTS OPACIFIANTS DE FAIBLE VISCOSITE

(30) Priorität: 29.03.1995 DE 19511572
(43) Veröffentlichungstag der Anmeldung: 14.01.1998
(73) Patentinhaber: Cognis Deutschland GmbH, 40589 Düsseldorf (DE)
(72) Erfinder: BAUMÖLLER, Guido, D-40597 Düsseldorf (DE); WADLE, Armin, D-40724 Hilden (DE); ANSMANN, Achim, D-40699 Erkrath (DE); TESMANN, Holger, D-41363 Jüchen (DE); FÖRSTER, Thomas, D-40699 Erkrath (DE)
(86) Internationale Anmeldenummer: EP9601197
(87) Internationale Veröffentlichungsnummer: WO9630476

(56) Entgegenhaltungen:
- EP-A- 0 510 870
- EP-A- 0 569 843
- WO-A-94/24248
- US-A- 4 623 471
- US-A- 4 767 617
- PATENT ABSTRACTS OF JAPAN vol. 018, no. 222 (C-1193), 21.April 1994 & JP,A,06 017088 (KAO CORP), 25.Januar 1994,

## Beschreibung

### Gebiet der Erfindung

Die Erfindung betrifft Trübungsmittel mit einem Gehalt an Wachskörpern, sowie einer Mischung zweier Emulgatoren unterschiedlichen HLB-Wertes.

### Stand der Technik

Bei der Formulierung einer Vielzahl von oberflächenaktiven Haushaltsprodukten, wie beispielsweise Geschirrspülmitteln oder Haarshampoos, wird besonderer Wert darauf gelegt, daß die Produkte möglichst klar vorliegen und auch im Verlauf der Lagerung nicht austrüben. In anderen Fällen werden für den gleichen Anwendungszweck Produkte gewünscht, die trübe sind und dabei einen Glimmereffekt, den sogenannten "Perlglanz" zeigen. Eine dritte Gruppe von Produkten wird mit einer nichtglänzenden Weißtrübung hergestellt, wobei sogenannte Trübungsmittel zum Einsatz gelangen. Trübungsmittel stellen feinteilige Polymer- bzw. Feststoffdispersionen dar, die neben Wasser und/oder einem Polyol - beispielsweise Glycerin - im wesentlichen nur noch einen Wachskörper und einen geeigneten Emulgator enthalten. An derartige Trübungsmittel werden die verschiedenartigsten Anforderungen gestellt. Ein besonderes Augenmerk liegt beispielsweise auf dem Feststoffgehalt, der einerseits so hoch wie möglich sein sollte, um Lagerkosten einzusparen, andererseits aber niedrig genug sein muß, um ein einwandfreies rheologisches Verhalten des Mittels zu ermöglichen. Von besonderer Wichtigkeit ist zudem, daß die Dispersionen sehr feinteilig sind, so daß eine allmähliche Sedimentation verhindert wird; zudem soll eine Weißtrübung und kein Perlglanz entstehen.

Die komplexe Aufgabe der Erfindung hat demnach darin bestanden, Trübungsmittelkonzentrate auf Basis von Wachskörpern zur Verfügung zu stellen, die hochkonzentriert, aber niedrigviskos sind, in wäßrigen Tensidlösungen eine Weißtrübung hervorrufen und infolge ihrer Feinteiligkeit ausreichend lagerstabil sind.

### Beschreibung der Erfindung

Gegenstand der Erfindung sind niedrigviskose Trübungsmittelkonzentrate, die sich dadurch auszeichnen, daß sie - bezogen auf die Mittel -
(a) 10 bis 30 Gew.-% Wachskörper
(b) 5 bis 15 Gew.-% Zuckertenside und
(c) 1 bis 5 Gew.-% Fettsäurepartialglyceride
mit der Maßgabe enthalten, daß die Komponenten (b) und (c) im Gewichtsverhältnis 15 : 1 bis 8 : 1 vorliegen.

Überraschenderweise wurde gefunden, daß nur bei Einsatz von Mischungen aus hydrophilen und hydrophoben nichtionischen Tensiden als Emulgatorsystem besonders feinteilige und niedrigviskose Produkte erhalten werden, die beim Einbringen in Tensidlösungen anstelle des erwarteten Periglanzes die gewünschte Weißtrübung hervorrufen.

### Wachskörper

Die Auswahl der Wachskörper, die die Komponente (a) bilden, ist an sich unkritisch. Typische Beispiele sind Alkylenglycolfettsäureester, Wachsester, gehärtete Triglyceride, gesättigte Fettalkohole mit 16 bis 18 Kohlenstoffatomen, Ethylenoxid-Addukte an Fettsäuren mit 16 bis 18 Kohlenstoffatomen und/oder Paraffinwachse. In einer bevorzugten Ausführungsform der Erfindung werden als Wachskörper Alkylenglycolfettsäureester der Formel **(I)** eingesetzt,

**R**^{**1**}**COO-[A]-OR**^{**2**} **(I)**

in der R¹CO für einen aliphatischen Acylrest mit 6 bis 22 Kohlenstoffatomen und 0 und/oder 1, 2 oder 3 Doppelbindungen, R² für R¹CO oder eine Hydroxylgruppe und A für eine lineare oder verzweigte, gegebenenfalls hydroxysubstituierte Alkylengruppe mit 2 bis 5 Kohlenstoffatomen steht. Vorzugsweise handelt es sich bei diesen Wachsen um Ester des Ethylenglycols oder Propylenglycols mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt ist der Einsatz von Ethylenglycoldistearat. Die Einsatzmenge der Alkylenglycolfettsäureester kann 10 bis 30 und vorzugsweise 15 bis 25 Gew.-% bezogen auf das Konzentrat betragen.

### Alkyl- und/oder Alkenyloligoglykoside

Als hydrophile Zuckertenside der Gruppe (b), die sich dadurch auszeichnen, daß sie einen HLB-Wert oberhalb von 10 aufweisen, kommen beispielsweise Alkyl- und Alkenyloligoglykoside in Betracht. Hierbei handelt es sich um bekannte Stoffe, die der Formel **(II)** folgen,

**R**^{**3**}**O-[G]**_{**p**} **(II)**

in der R³ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht, und die nach den einschlägigen Verfahren der präparativen organischen Chemie erhalten werden können. Stellvertretend für das umfangreiche Schrifttum sei hier auf die Schriften **EP 0301298 A1** und **WO 90/03977** verwiesen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel **(II)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/ oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R³ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R³ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, die wie oben beschrieben erhalten werden können. Bevorzugt sind Alkyloligoglucoside auf Basis von gehärtetern C_{12/14}-Kokosalkohol mit einem DP von 1 bis 3.

### Fettsäure-N-alkylpolyhydroxyalkylamide

Als weitere hydrophile Zuckertenside kommen Fettsäure-N-alkylpolyhydroxyalkylamide der Formel **(III)** in Frage, 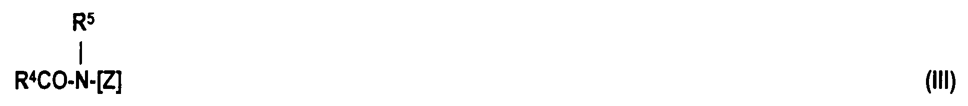 in der R⁴CO für einen aliphatischen Acylrest mit 6 bis 22 Kohlenstoffatomen, R⁵ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424**, **US 2,016,962** und **US 2,703,798** sowie die Internationale Patentanmeldung **WO 92/06984** verwiesen. Eine Übersicht zu diesem Thema von H.Kelkenberg findet sich in **Tens.Surf.Det. 25**, **8 (1988)**. Vorzugsweise leiten sich die Fettsäure-N-alkylpolyhydroxyalkylamide von reduzierenden Zukkern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel **(IV)** wiedergegeben werden:

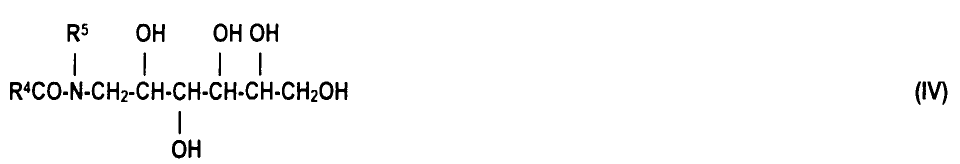

Vorzugsweise werden als Fettsäure-N-alkylpolyhydroxyalkylamide Glucamide der Formel **(IV)** eingesetzt, in der R⁵ für Alkylrest mit 1 bis 4 Kohlenstoffatomen steht und R⁴CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel **(IV)**, die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

Die Einsatzmenge der hydrophilen Zuckertenside kann vorzugsweise 7,5 bis 12,5 Gew.-% bezogen auf das Konzentrat betragen.

### Fettsäurepartialglyceride

Fettsäurepartialglyceride, die als hydrophobe Emulgatorkomponente (c) einen HLB-Wert unterhalb von 10 aufweisen, stellen Mono- oder Diglyceride sowie technische Gemische beider dar und folgen der Formel **(V)**, 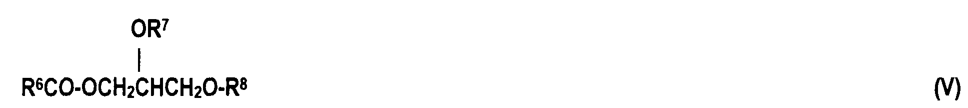 in der R⁶CO für einen aliphatischen Acylrest mit 6 bis 22 Kohlenstoffatomen und 0 und/oder 1, 2 oder 3 Doppelbindungen und R⁷ und R⁸ unabhängig voneinander für Wasserstoff oder R⁶CO stehen, mit der Maßgabe, daß mindestens einer der beiden Reste R⁷ und R⁸ Wasserstoff bedeuten. Typische Beispiele sind Mono- und Diester des Glycerins mit Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Aus anwendungstechnischer Sicht besonders bevorzugt sind technische Monoglyceride auf Basis von Laurinsäure, Ölsäure oder Stearinsäure sowie Kokos- oder Talgfettsäure. Die Einsatzmenge der hydrophoben Fettsäurepartialglyceride kann vorzugsweise 1,5 bis 3 Gew.-% bezogen auf das Konzentrat betragen.

### Gewerbliche Anwendbarkeit

Die erfindungsgemäßen Trübungsmittelkonzentrate zeichnen sich durch eine niedrige Viskosität, gute Fließ- und Pumpeigenschaften sowie eine besondere Feinteiligkeit der Kristalle in der Dispersion aus. Demzufolge besteht ein weiterer Vorteil dieser Mittel in ihrer hohen Stabilität gegen Sedimentieren bei längerer Lagerung. In Mengen von 0,5 bis 5, vorzugsweise 1 bis 2 Gew.-% - bezogen auf die wäßrigen oberflächenaktive Mittel wie beispielsweise manuelle Geschirrspülmittel, Haarshampoos oder Duschbäder und dergleichen - eingesetzt, rufen die Mittel eine dauerhafte und gleichmäßige Weißtrübung hervor, ohne daß dabei Perlglanz erzeugt wird.

### Tenside

Die Auswahl der Tenside, in deren wäßrigen Lösungen die erfindungsgemäßen Mittel eine Weißtrübung hervorrufen, ist unkritisch, da das Eintreten des Effektes weitgehend unabhängig von der Natur der Tensidkomponente ist. Demzufolge können die Trübungsmittel in wäßrigen Lösungen von anionischen, nichtionischen, kationischen und/oder amphoteren bzw. zwitterionischen Tensiden eingesetzt werden. Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acylglutamate, Acyltartrate, Alkyloligoglucosidsulfate, Proteinfettsäurekondensate (insbesondere pflanzliche Produkte auf Sojabasis) und Alkyl(ether)phosphate. Sofern die anionischen Tenside Polyglycoletherketten enthalten, können sie eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolygylcolether, Fettaminpolyglycolether, alkoxylierte Triglyceride, Mischether bzw. Mischformale, Proteinhydrolysate (insbesondere pflanzliche Produkte auf Sojabasis) Polyolfettsäureester, Zuckerester, Sorbitanester und Polysorbate. Sofern die nichtionischen Tenside Polyglycoletherketten enthalten, können sie eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S.54-124** oder **J.Falbe (ed.), "Katalysatoren, Tenside und Mineralöladditive", Thieme Verlag, Stuttgart, 1978, S.123-217** verwiesen.

### Ölkörper

Die erfindungsgemäßen Konzentrate können des weiteren Ölkörper enthalten, wie z.B. Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₁₆-C₁₈-Fettalkoholen, Ester von linearen C₁₀-C₁₈-Fettsäuren mit verzweigten Alkoholen, insbesondere 2- Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate und/oder Dialkylether. Die wäßrigen Tensidlösungen können unmittelbar oder nach Abmischungen mit weiteren Rezepturbestandteilen mit den Trübungsmitteln versetzt werden und dabei ihrerseits einen Feststoffgehalt von 1 bis 60, vorzugsweise 5 bis 35 Gew.-% - bezogen auf die Lösungen - aufweisen.

### Beispiele

Die Viskosität der verschiedenen Trübungsmittelkonzentrate wurde nach der Brookfield-Methode (23°C, Spindel 5, 10 Upm) ermittelt und die Feinteiligkeit unter dem Mikroskop visuell auf einer Skala von 1 = sehr feine Kristalle bis 5 = grobe Kristalle beurteilt. Die Ergebnisse sind in Tabelle 1 zusammengefaßt (Prozentangaben als Gew.-%):

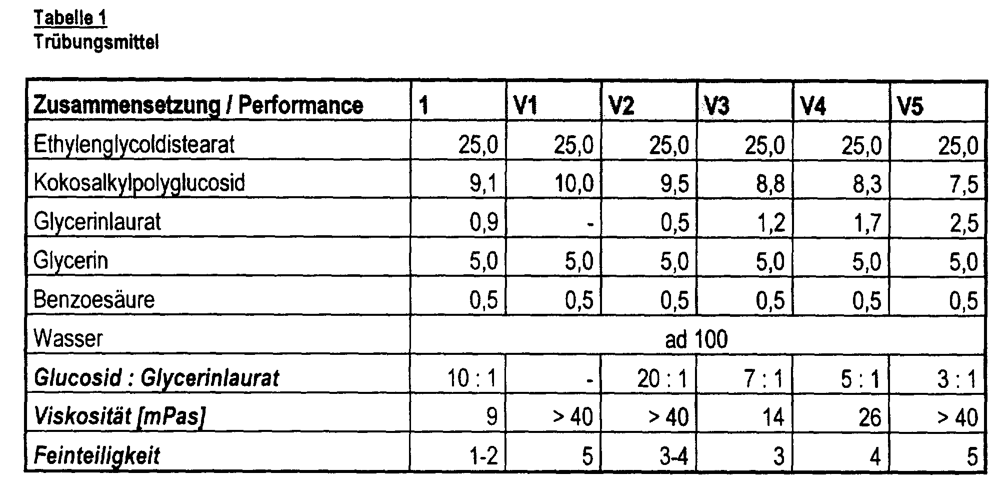

## Patentansprüche

1. Niedrigviskose Trübungsmittelkonzentrate, **dadurch gekennzeichnet**, daß sie - bezogen auf die Mittel -
(a) 10 bis 30 Gew.-% Wachskörper
(b) 5 bis 15 Gew.-% Zuckertenside und
(c) 1 bis 5 Gew.-% Fettsäurepartialglyceride
mit der Maßgabe enthalten, daß die Komponenten (b) und (c) im Gewichtsverhältnis 15 : 1 bis 8 : 1 vorliegen.

2. Konzentrate nach Anspruch 1, **dadurch gekennzeichnet**, daß sie Wachskörper enthalten, die ausgewählt sind aus der Gruppe, die gebildet wird von Alkylenglycolfettsäureestern, Wachsestern, gehärteten Triglyceriden, gesättigten Fettalkoholen mit 16 bis 18 Kohlenstoffatomen, Ethylenoxidaddukten an Fettsäuren mit 16 bis 18 Kohlenstoffatomen und/oder Paraffinwachsen.

3. Konzentrate nach den Ansprüchen 1 und 2, **dadurch gekennzeichnet**, daß sie als Komponente (a) Alkylenglycolfettsäureester der Formel **(I)** enthalten,
**R**^{**1**}**COO-[A]-OR**^{**2**} **(I)**
in der R¹CO für einen aliphatischen Acylrest mit 6 bis 22 Kohlenstoffatomen und 0 und/oder 1, 2 oder 3 Doppelbindungen, R² für R¹CO oder Wasserstoff und A für eine lineare oder verzweigte, gegebenenfalls hydroxysubstituierte Alkylengruppe mit 2 bis 5 Kohlenstoffatomen steht.

4. Konzentrate nach den Ansprüchen 1 bis 3, **dadurch gekennzeichnet**, daß sie als Komponente (b) Alkyl- und/oder Alkenyloligoglykoside der Formel **(II)** enthalten,
**R**^{**3**}**O-[G]**_{**p**} **(II)**
in der R³ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

5. Konzentrate nach den Ansprüchen 2 bis 5, **dadurch gekennzeichnet**, daß sie als Komponente (b) Fettsäure-N-alkylpolyhydroxyalkylamide der Formel **(III)** enthalten, 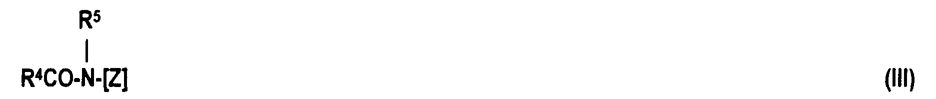 in der R⁴CO für einen aliphatischen Acylrest mit 6 bis 22 Kohlenstoffatomen, R⁵ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

6. Konzentrate nach den Ansprüchen 2 bis 5, **dadurch gekennzeichnet**, daß sie als Komponente (c) Fettsäurepartialglyceride der Formel **(V)** enthalten, 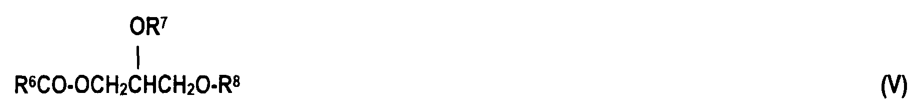 in der R⁶CO für einen aliphatischen Acylrest mit 6 bis 22 Kohlenstoffatomen und 0 und/oder 1, 2 oder 3 Doppelbindungen und R⁷ und R⁸ unabhängig voneinander für Wasserstoff oder R⁶CO stehen, mit der Maßgabe, daß mindestens einer der beiden Reste R⁷ und R⁸ Wasserstoff bedeuten.

## Claims

1. Low-viscosity opacifier concentrates based on waxes with a solids content of 40 to 60% by weight, characterized in that they contain mixtures of
(a) sugar surfactants and
(b) fatty acid partial glycerides
in a ratio by weight of 15:1 to 8:1 as emulsifiers.

2. Concentrates as claimed in claim 1, characterized in that they contain waxes selected from the group consisting of alkylene glycol fatty acid esters, wax esters, hydrogenated triglycerides, saturated fatty alcohols containing 16 to 18 carbon atoms, ethylene oxide adducts with fatty acids containing 16 to 18 carbon atoms and/or paraffin waxes.

3. Concentrates as claimed in claims 1 and 2, characterized in that they contain alkylene glycol fatty acid esters corresponding to formula **(I)**:
**R**^{**1**}**COO-[A]-OR**^{**2**} **(I)**
in which R¹CO is an aliphatic acyl radical containing 6 to 22 carbon atoms and 0 and/or 1, 2 or 3 double bonds, R² has the same meaning as R¹CO or is hydrogen and A is a linear or branched, optionally hydroxy-substituted alkylene group containing 2 to 5 carbon atoms.

4. Concentrates as claimed in claims 1 to 3, characterized in that they contain alkyl and/or alkenyl oligoglycosides corresponding to formula **(II)**:
**R**^{**3**}**O-[G]**_{**p**} **(II)**
where R³ is an alkyl andlor alkenyl radical containing 4 to 22 carbon atoms, G is a sugar unit containing 5 or 6 carbon atoms and p is a number of 1 to 10, as component (a).

5. Concentrates as claimed in claims 2 to 5, characterized in that they contain fatty acid-N-alkyl polyhydroxyalkylamides corresponding to formula **(III)**: 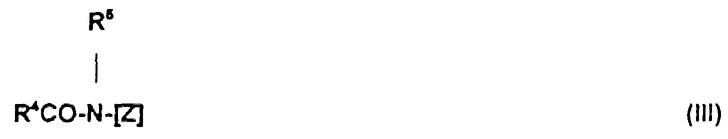 in which R⁴CO is an aliphatic acyl radical containing 6 to 22 carbon atoms, R⁵ is hydrogen, an alkyl or hydroxyalkyl radical containing 1 to 4 carbon atoms and [Z] is a linear or branched polyhydroxyalkyl radical containing 3 to 12 carbon atoms and 3 to 10 hydroxyl groups,
as sugar surfactants.

6. Concentrates as claimed in claims 2 to 5, characterized in that they contain fatty acid partial glycerides corresponding to formula **(V)**: 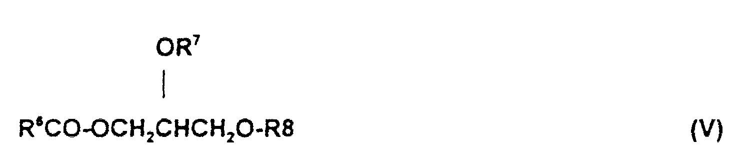 where R⁶CO is an aliphatic acyl radical containing 6 to 22 carbon atoms and 0 andlor 1, 2 or 3 double bonds and R⁷ and R⁸ independently of one another represent hydrogen or have the same meaning as R⁶CO, with the proviso that at least one of the two substituents R⁷ and R⁸ is hydrogen.

7. Concentrates as claimed in claims 1 to 6, characterized in that they contain oils as further constituents.

## Revendications

1. Concentrés d'agents opacifiants faiblement visqueux,
caractérisés en ce qu'
ils contiennent - rapportés à l'agent :
a) de 10 à 30 % en poids de composé cireux,
b) de 5 à 15 % en poids d'agent tensioactif dérivé de sucre, et
c) de 1 à 5 % en poids de glycérides partiels d'acide gras, avec la réserve que les composants b) et c) se présentent dans un rapport en poids de 15 : 1 à 8 : 1.

2. Concentrés selon la revendication 1,
caractérisés en ce qu'
ils contiennent des composés cireux qui sont choisis dans le groupe qui est formé d'esters d'alkylèneglycol d'acide gras, d'esters de cires, de triglycérides durcis, d'alcools gras saturés ayant de 16 à 18 atomes de carbone, des produits d'additions de l'oxyde d'éthylène sur des acides gras ayant de 16 à 18 atomes de carbone et/ou des cires de paraffine.

3. Concentrés selon les revendications 1 et 2,
caractérisés en ce qu'
ils renferment comme composant a) des esters d'alkylèneglycol d'acide gras de formule (I) :
**R**^{**1**}**COO-[A]-OR**^{**2**} **(I)**
dans laquelle R¹CO représente un radical acyle aliphatique ayant de 6 à 22 atomes de carbone, et 0 et/ou 1, 2 ou 3 double liaisons, R² représente R¹CO ou de l'hydrogène, et A représente un groupe alkylène linéaire ou ramifié, éventuellement substitué par un hydroxy, ayant de 2 à 5 atomes de carbone.

4. Concentrés selon les revendications 1 à 3,
caractérisés en ce qu'
ils renferment comme composants b) des alkyl- et/ou des alkényloligoglycosides de formule (II) :
**R**^{**3**}**O-[G]**_{**p**} **(II)**
dans laquelle R³ représente un radical alkyle et/ou alkényle ayant de 4 à 22 atomes de carbone, G représente un radical de sucre ayant 5 ou 6 atomes de carbone et p représente un nombre de 1 à 10.

5. Concentrés selon les revendications 2 à 5,
caractérisés en ce qu'
ils renferment comme composant b) des N-alkylpolyhydroxyalkylamides d'acide gras de formule (III) : 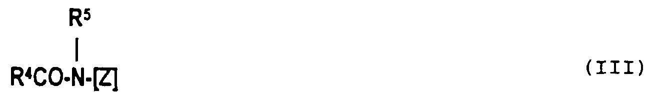 dans laquelle R⁴CO représente un radical acyle aliphatique ayant de 6 à 22 atomes de carbone, R⁵ représente un radical alkyle ou hydroxyalkyle ayant de 1 à 4 atomes de carbone, et [Z] représente un radical polyhydroxyalkyle linéaire ou ramifié ayant de 3 à 12 atomes de carbone, et de 3 à 10 groupes hydroxyle.

6. Concentrés selon les revendications 2 à 5,
caractérisés en ce qu'
ils renferment comme composants c) des glycérides partiels d'acide gras de formule (V) : 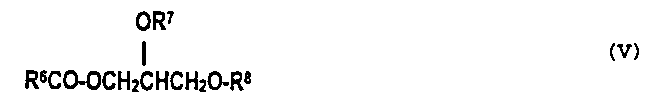 dans laquelle R⁶CO représente un radical acyle aliphatique ayant de 6 à 22 atomes de carbone et 0 et/ou 1, 2, ou 3 double liaisons et R⁷ et R⁸, indépendamment l'un de l'autre, représentent de l'hydrogène ou R⁶CO, avec la réserve qu'au moins un des deux radicaux R⁷ et R⁸ signifie de l'hydrogène.
